# EUROPEAN PATENT SPECIFICATION

(11) **EP 1 766 028 B1**
(45) Date of publication and mention of the grant of the patent: **02.03.2011**
(21) Application number: 05741945.9
(22) Date of filing: 19.05.2005
(51) Int. Cl.: C12N 15/81, C12P 7/08, C12N 1/18, C12R 1/865, C07K 1/00

(54) **ETHANOL PRODUCTIVITIES OF SACCHAROMYCES CEREVISIAE STRAINS IN FERMENTATION OF DILUTE-ACID HYDROLYZATES DEPEND ON THEIR FURAN REDUCTION CAPACITIES**
ETHANOLPRODUKTIVITÄTEN VON SACCHAROMYCES-CEREVISIAE-STÄMMEN BEI DER VERGÄRUNG VON VERDÜNNTE-SÄURE-HYDROLYSATEN HÄNGEN VON IHREM FURANREDUKTIONSVERMÖGEN AB
LA PRODUCTION D'ETHANOL PAR DES SOUCHES DE SACCHAROMYCES CEREVISIAE EN FERMENTATION D'HYDROLYSATS ACIDES DILUES DEPEND DE LEUR CAPACITE A REDUIRE LE FURANE

(30) Priority: 19.05.2004 SE 0401303
(43) Date of publication of application: 28.03.2007
(73) Proprietor: Scandinavian Technology Group AB, 223 70 Lund (SE)
(72) Inventor: NILSSON, Anneli, S-217 49 Malmö (SE); LIDÉN, Gunnar, S-224 71 Lund (SE); GORWA-GRAUSLUND, Marie-Francoise, S-211 27 Malmö (SE); HAHN-HÄGERDAL, Bärbel, S-223 61 Lund (SE); MODIG, Carl Tobias, 244 39 Kävlinge (SE); MOREIRA DE ALMEIDA, Joao Ricardo, 222 40 Lund (SE)
(74) Representative: Stenbäck, Maria Elisabeth
(86) International application number: PCT/SE2005/000738
(87) International publication number: WO 2005/111214

(56) References cited:
- LARROY C. ET AL: 'Characterization of the Saccharomyces cerevisiae YMR318C (ADH6) gene product as a broad specificity NADPH-dependent alcohol dehydrogenase: relevance in aldehyde reduction.' BIOCHEM.J. vol. 361, 2002, pages 163 - 172, XP002990994
- DICKINSON J.R. ET AL: 'The Catabolism of Amino Acids to Long Chain and Complex Alcohols in Saccharomyces cerevisiae.' THE JOURNAL OF BIOLOGICAL CHEMISTRY. vol. 278, no. 10, 2003, pages 8028 - 8034, XP002990995
- MARTIN C. ET AL: 'Comparison of the resistance of industrial and laboratory strains of Saccharomyces and Zygosaccharomyces to lignocellulose-derived fermentation inhibitors.' ENZYME AND MICROBIAL TECHNOLOGY. vol. 32, 2003, pages 386 - 395, XP002990996
- LARSSON S. ET AL: 'Ethanol from lignocellulose-Fermentation Inhibitors, Detoxification and Genetic Engineering of Saccharomyces cerevisiae for Increased Resistance.' APPLIED MICROBIOLOGY., [Online] 1971, XP002990997 Retrieved from the Internet: <URL:URL:http://theses.lub.lu.se/postgrad/s earch.tkl?field_query=pubid&queryl=tec_399& rec...>
- HORVATH I.S. ET AL: 'Effects of Furfural on the Respiratory Metabolism of Saccharomyces cerevisiae in Glucose-Limited Chemostats.' APPLIED AND ENVIRONMENTAL MICROBIOLOGY. vol. 69, no. 7, July 2003, pages 4076 - 4086, XP002990998
- WAHLBOM C.F. ET AL: 'Furfural, 5-Hydroxymethyl Furfural, and Acetoin Act as External Electron Acceptors During Anaerobic Fermentation of Xylose in Recombinant Saccharomyces cerevisiae.' BIOTECHNOLOGY AND BIOENGINEERING. vol. 78, no. 2, April 2002, pages 172 - 178, XP002990999
- TAHERZADEH M.J. ET AL: 'Physiological effects of 5-hydroxymethylfurfural on Saccharomyces cerevisiae.' APPL MICROBIOL BIOTECHNOL. vol. 53, 2000, pages 701 - 708, XP002991000
- WAHLBOM C.F. ET AL: 'Generation of the improved recombinant xylose-utilizing Saccharomyces cerevisiae TMB 3400 by random mutagenesis and physiological comparison with Pichia stipitis CBS 6054.' FEMS YEAST RESEARCH. vol. 3, 2003, pages 319 - 326, XP002980002
- KUYPER M. ET AL: 'Minimal metabolic engineering of Saccharomyces cerevisiae for efficient anaerobic xylose fermentation: a proof of principle.' FEMS YEAST RESEARCH. vol. 4, 2004, pages 655 - 664, XP002312911

## Description

### Technical field

The present invention relates to a *Saccharomyces cerevisiae* strain being able to grow and produce ethanol from lignocellulosic hydrolysates in the presence of inhibiting compounds and substances, in particular furfural and derivatives thereof while reducing such compounds, and in particular it relates to a strain that is able to produce ethanol in a fed-batch or continuous production system. The invention further relates to the use of Saccharomyces cerevisiae strain for growing and producing ethanol from lignocellulosic hydrolysates.

### Background of the invention

Because of Its low net contribution to the production of carbon dioxide, ethanol produced from renewable resources, such as lignocellulose, is considered an attractive alternative for partly replacing fossil fuels (1). Many sources of lignocellulosic materials (e.g. wood, forest residues and agricultural residues) can potentially be used for ethanol production (2). Prior to fermentation, however, the cellulose and the hemicellulose in the lignocellulose must be converted to monomeric sugars by a combination of physical (e.g. grinding, steam explosion), chemical (e.g. dilute acid) and perhaps also enzymatic treatments (2). In addition to monomeric sugars also a number of other compounds are formed during these processes, several of which are potent inhibitors. Examples of such compounds are carboxylic acids, furans and phenolic compounds (3, 4, 5, 6, 7). The microorganism used for fermentation of hydrolyzates should consequently exhibit three characteristics: a) it should have high ethanol tolerance, b) it should be resistant to inhibitors found in the hydrolyzate and c) it should have a broad substrate utilization range, since the hydrolyzate contains several different sugars. The quantitively most important sugars in hydrolyzate from spruce are glucose, mannose and xylose (6).

Due to Its high ethanol yield, high specific productivity and high ethanol tolerance, *Saccharomyces cerevisiae* is the preferred microorganism for conversion of hydrolyzate to ethanol. It has also been shown that this yeast species is more tolerant to inhibitors such as acetic acid, furfural and 5-hydroxy-methyl furfural (HMF) than several other potential production microorganisms (8). The tolerance to, in particular, many of the aldehyde compounds can most likely be explained by a bioconversion of these compounds by the yeast to, in general, the less inhibitory corresponding alcohols. It is for instance known that S, *cerevisiae* converts furfural into the less inhibiting compound furfuryl alcohol (9, 10). With respect to sugar utilization, *S. cerevisiae* efficiently converts both glucose and mannose into ethanol, but is unable to convert xylose into ethanol. Other yeast species, e.g. *Pichia stipitis* and *Candida shehatae* are able to convert xylose into ethanol. However, these yeasts have a relatively low ethanol and inhibitor tolerance, and, furthermore, require microaerobic conditions in order to give a high productivity (8, 11). Work has consequently been made to genetically engineer *S. cerevisiae* in order to obtain xylose-fermenting capacity. In the xylose-metabolizing yeasts, xylose is channeled into the pentose phosphate pathway (PPP) in a three-step process. Xylose is first converted to xylitol by a xylose reductase (XR). Xylitol is then oxidized to xylulose by xylitol dehydrogenase (XDH), and finally, xylulose is phosphorylated to xylulose 5-phosphate by xylulose kinase (XK) (12). The first two enzymes are lacking in *S. cerevisiae.* Furthermore, the activity of XK in *S. cerevisiae* has been shown to be low (13), which has been suggested to limit the consumption rate in *S. cerevisiae* strains expressing XR and XDH (14). However, strain background appears to be important for the effect of XK (15, 16). In the present work, a genetically modified xylose-utilizing strain of S, *cerevisiae* was studied: TMB3006 (17). This strain express the heterologous genes *XYL1* and *XYL2* (encoding the enzymes XR and XDH, respectively) from *P. stipitis,* and overexpress the native gene *XKS1* (encoding XK).

It has previously been shown that strongly inhibiting dilute-acid hydrolyzates, not fermentable in a batch process, can be fermented by *S. cerevisiae* without prior detoxification in a fed-batch operation. This, however, requires a carefully controlled hydrolyzate feed-rate (18, 19, 20). The most likely explanation for the success of fed-batch operation is that inhibitors are maintained at low levels because of their conversion to less toxic compounds. In the development of a closed-loop control strategy for fed-batch fermentation, the *S. cerevisiae* strain CBS 8066 (21) was used. It is known that different strains of *S. cerevisiae* show significant differences in fermentative capacity and inhibitor tolerance in batch cultivation. In the work by Carlos et. al. (22) significant differences in the ethanol productivity of several strains were found in batch cultivations with different levels of an inhibitor cocktail in a synthetic media. Only three out of 13 tested strains produced ethanol in batch fermentation with the highest level of the inhibitor cocktail. Not only the performance in batch culture, but even more so the performance in fed-batch culture is important for selection of a suitable strain. Besides the possibility to control the level of several potential inhibitors, the fed-batch operation offers another advantage in comparison to batch operation, which is the possibility of a parallel uptake of several sugars. The reason is that the concentration of sugars can be maintained at a low level by controlling the feed rate. In this way saturation of uptake systems (or saturation of the glycolytic flux) can be avoided, making co-fermentation of different sugars possible. In S, *cerevisiae,* xylose is believed to be transported by the same uptake system as glucose, however, with a much lower affinity (23). It is also known that a concomitant uptake of glucose increases the xylose consumption rate (24). One may therefore anticipate that a higher specific conversion rate xylose in hydrolyzates can be obtained in fed-batch cultivations.

Larroy, C., M. R. Fernandez, G. E, X. Pares, and J. A. Blosca. 2002. Characterization of the Saccharomyces cerevisiae YMR318C (ADH6) gene product as a broad specifity NADPH-dependant alcohol dehydrogenase: relevance in aldehyde reduction. Biochem J. 361:163-172 (38) have characterised the enzyme ADHVI and tested its kinetics for several substrates, primarily aliphatic and aromatic aldehydes. In their work the authors have primarily concentrated on the aromatic aldehydes (cinnamaldehyde and veratraldehyde). The authors suggest that ADHVI may give the yeast the opportunity to survive in ligninolytic environments where products derived from lignin biodegradation may be available. However, no tests have been made concerning the ability of ADHVI to use HMF as a substrate, HMF being a carbohydrate derived product. Furthermore, the paper does not discuss, or experimentally investigate, the potential role of ADHVI in protection against or conversion of Inhibitors resulting from breakdown of the carbohydrates such as cellulose and/or hamicellulose. Larroy only discloses transformation of the yeast strain BJ2168 with the YMR318Cgene.

Dickinson, J. R., L. Eshantha, J. Salgado, and M. J. E. Hewilns. 2003. The catabolism of amino adds to long chain complex alcohols in Saccharomyces cerevisiae. The Journal of Biological Chemistry 278:8028-8034. have studied the final step in the formation of long chain or complex alcohols in S. cerevisiae. They conclude that any of one of the six alcohol dehydrogenases (encoded by *ADH1, ADH2, ADH3, ADH4, ADH5* or *SFA1)* is sufficient for the final stage of long chain or complex alcohol formation. Mutant strains were grown on single amino acids and fusel alcohol formation was measured. No measurements of enzyme activities in lysates nor any assessment of co-factor requirements were made. Importantly, the gene *ADH6* was not at all studied, since it was regarded unlikely by the authors that an NADPH-dependent enzyme would be involved in fusel alcohol formation. The paper by Dickinson et al is therefore completely unrelated to the conversion of HMF and furfural, and is completely unrelated to any conversion catalyzed by the gene product of *ADH6.*

Martin, C., and L. J. Jönsson. 2003. Comparison of the resistance of industrial and laboratory strains of Saccharomyces and Zygosaccharomyces to lignocellulose-derived fermentation inhibitors. Enzyme and Microbial Technology 32:386-395., (3) have performed a comparison between 13 different yeast strains with respect to their resistance to lignocellulose-derived fermentation inhibitors. The strains are exposed to the following Inhibitors: formic acid, acetic acid, furfural, HMF, cinnamic acid and coniferyl aldehyde. It is concluded that there is a big difference between the strains ability to tolerate and convert the inhibitors. However, no mechanistic investigations on the enzymes responsible for the conversion are presented or discussed in the paper. Specifically, there is no referral to either the gene product of *ADH6* nor co-factor dependence In the reduction, made in the paper.

In the present work, different strains of *S. cerevisiae* were characterized in both batch and fed-batch fermentations of dilute-acid hydrolyzates. A total of four different strains were studied: CBS 8066, commercial bakers yeast, TMB3000, and TMB3006. The specific ethanol productivity, specific growth rate, consumption rates of monosaccharides, cell viability and furan reduction activities were determined. The results suggest that the furan reducing capacity is a key factor behind tolerance to lignocellulosic hydrolyzates.

### Materials and Methods

### Strains and medium used

The four strains of *Saccharomyces cerevisiae* used are given in Table 1. The strains were maintained on agar plates with the following composition: 10 g/l yeast extract, 20 g/l soy peptone, 20 g/l agar and 20 g/l glucose. Inoculum cultures were grown in 300 ml cotton plugged E-flasks with 100 ml of synthetic media according to Taherzadeh et. al. (25) with 15 g/l glucose as carbon and energy source. The inoculum cultures were grown for 24 h at 30°C and with a shaker speed of 150 rpm before 20 ml was added to the fermentor to start the cultivation.

### Fermentation conditions

### Hydrolyzate medium

The hydrolyzate used was produced from forest residue, originating mainly from spruce, in a two-stage dilute-acid hydrolysis process using sulphuric acid as the catalyst (19). The hydrolyzates obtained from the two stages were mixed and stored at 8°C until used. The composition of the hydrolyzate is given in Table 2.

### Initial batch cultivation

Fermentation experiments were performed in a 3.3 1 BioFlo III bioreactor (New Brunswick Scientific, Edison, NJ, USA). The stirring rate was 400 rpm and the fermentor was continuously sparged with 600 or 1000 ml/min nitrogen gas (oxygen content Larsson, Simona et al 1971. Ethanol from Lignocellulose-Fermentation inhibitors, detoxification and genetic engineering of Saccharomyces Cerevisiae for increased resistance, Applied Microbiology. Larsson et al have studied different detoxification methods, wherein the most efficient method was the anion exchange chromatography.

Horváth, Ilona Sárvári et al. 2003. Effects of furfural on the respiratory metabolism of Saccharomyces cerevisiae in Glucose-Limited Chemostats. Applied and Environmental Microbiology, vol. 69, pg 4076-4086. Horvàth et al have studied the physiological effects of furfural on the yeast Saccharomyces cerevisiae during respiratory growth on glucose.

Wahlbom, C. Fredrik et al. Furfural, 5-hydroxymethyl furfural and acetoin act as an external electron acceptors during anaerobic fermentation of xylose in recombinant Saccharomyces cerevisiae, Biotechnology and Bioengineering, April 2002, Vol. 78, No. 2, p. 172-178. Wahlbom et al have studied the influence of external electron acceptors (acetoin, acetaldehyde, furfural and HMF) on the fermentation of xylose in a lab medium using a recombinant Saccharomyces cerevisiae, which harbours genes for xylose reductase, xylitol dehydrogenase and xylulokinase. It is shown that furfural (but not HMF) as external electron acceptor is leading to reduced xylitol fermentation. The experiments have not been performed in a lignocellulosic hydrolysate.

Taherzadeh, M.J, et al. Physiological effects of 5-hydroxymethylfurfural on Saccharomyces cerevisiae, Appl. Microbial, Technol, 2000, vol. 53, pg 701-708. Taherzadeh et al have studied the physiological effects of 5-hydroxymethylfurfural (HMF) on the Saccharomyces cerevisiae strain CBS 8066 in the presence and absence of furfural. Taherzadeh et al make a hypothesis that alcohol dehydrogenases convert HMF and furfural to its corresponding alcohols.

Wahlbom, C. Fredrik et a1. Generation of the improved recombinant xylose-utilizing Saccharomyces cerevisiae TMB 3400 by random mutagenesis and physiological comparison with Pichia stipitis CBS 6054, FEMS Yeast Research, 2003, Vol. 3, pg 319-326. Wahlbom et al disclose new improved recombinant xylose-utilizing Saccharomyces cerevisiae TMB3400. This piece of prior art does not discuss the influence the inhibitors HMF and furfural might have on the fermentation of the yeast strains.

Kuyper, M et al. Minimal metabolic engineering of Saccharomyces cerevisiae for efficient anaerobic xylose fermentation: a proof of principle. FEMS Yeast Research, 2004, Vol. 4, No. 6, pg 655-664. Kuyper et a1 disclose that minimal genetic engineering in the form of incorporation of the XylA gene in Saccharomyces cerevisiae is required to recruit a functional xylose metabolic pathway. This piece of prior art does not discuss the influence the inhibitors HMF and furfural might have on the fermentation of the yeast strains.

### Summary of the present invention

In one aspect the present invention relates to an ethanol producing *Saccharomyces cerevisiae* strain CEN.PK113-5D, being able to grow and produce ethanol from lignocelluosic hydrolysates comprising growth inhibiting compounds of the group furfural and 5-hydroxy-methyl furfural, in a batch, fed-bacth or continuous fermentation, which strain is unregulated and/or over expressed with regard to the ADH6 gene, wherein the alcoholdehydrogenase is NADPH dependent.
p. 4b

In another aspect the present invention relates to the use of an ethanol producing *Saccharomyces cerevisiae* strain for growing and producing ethanol from lignocellulosic hydrolysates comprising growth inhibiting compounds of the group furfural and 5-hydroxymethyl furfural, in a batch, fed-bacth or continuous fermentation, which strain is upregulated and/or over expressed with regard to the ADH6 gene, wherein the alcoholdehydrogenase is NADPH dependent.

In an embodiment of the invention said *Saccharomyces cerevisiae* strain is the *Saccharomyces cerevisiae* CEN.PK113-5D. In another embodiment said use of the *Saccharomyces cerevisiae* strain is for the reduction of furfural. guaranteed to be less than 5 ppm, ADR class2, 1(a), AGA, Sweden). The pH was maintained at 5.0 with 2.0 M NaOH. All experiments started with an initial batch phase in 1 l of synthetic media according to Taherzadeh et.al. (25) with 50 g glucose as carbon and energy source. However, the concentrations of media components other than glucose were tripled to compensate for the dilution during fed-batch operation. Hydrolyzate feeding was started at the depletion of the glucose, when the carbon evolution rate had decreased to less than 1 mmol/h.

### Batch and fed-batch fermentation

Two types of fermentation experiment were made for each strain. In the first type of experiment, 1.5 liter of the hydrolyzate was added to the reactor using the maximum feed rate of the medium pump (approximately 2 liters/h) after the initial batch cultivation. This is referred to as "batch" fermentation. The second type of experiment was a fed-batch experiment, in which the hydrolyzate feed rate was controlled using a step-response method developed by Nilsson et. al. (19). In short, the feed-rate was changed in a step-wise manner, in which the step increase was proportional to the derivative of the measured carbon dioxide evolution rate from the previous step. Feed rate control was obtained by controlling the frequency of a peristaltic pump (Watson-Marlow Alitea AB, Sweden). Also in these experiments, a total of 1.5 I of hydrolyzate was added.

### Xylose fermentation

Additional fed-batch experiments were made with the xylose-fermenting yeast (TMB3006) using low feed-rates (12.5 and 25 ml/h). The purpose of these experiments was to obtain low medium concentrations of glucose and mannose, expected to give an increased xylose uptake rate.

### Analyses

### Off-gas analysis

A gas monitor (model 1311, Brüel and Kjaer, Denmark) (described by Christensen et.al.) was used to measure the carbon dioxide evolution rate (CER). The gas analyzer had three channels for measurement of carbon dioxide, oxygen and ethanol in the off-gas from the reactor. The ethanol signal was calibrated against ethanol concentrations measured in the broth by HPLC, since it was assumed that the ethanol in the gas phase was in equilibrium with the ethanol in the broth. Calibration for oxygen and carbon dioxide was done using a gas containing 20.0% oxygen and 5.0% carbon dioxide.

### Biomass

A flow-injection-analysis (FIA) system (26) was used to measure biomass concentration in the reactor. This was done by measuring the optical density at 610 nm, at a frequency of 1 h⁻¹. After every fermentation the FIA-signal was calibrated against measured dry-weight. Duplicate 10 ml samples of the fermentation broth were centrifuged at 3000 rpm for 3 min in pre-weighted tubes. The cells were washed with distilled water, centrifuged again and dried over night at 105°C before they were weighted again. The dry weight was measured three times during each fermentation.

### Viability

Cell viability was measured as the ratio between colony forming units (CFU) and counted cell numbers three times during each fermentation. Samples were withdrawn from the fermentation broth and diluted to give a concentration of around 1000 cells/ml, and CFUs were determined from triplicate agar plates onto which 0.1 ml samples of diluted broth were spread. Cell numbers were calculated under microscope using a Bürker chamber. Prior to the calculation the samples were diluted 100 times.

### Metabolite concentrations

Samples for analysis of metabolite concentrations were taken regularly from the reactor. The samples were centrifuged and filtered trough 0.2 µm filters. The concentrations of glucose, mannose, xylose, galactose and arabinose were measured on an Aminex HPX-87P column (Bio-Rad, USA) at 80°C. The concentrations of ethanol, HMF, furfural, glycerol and acetic acid were measured on an Aminex HPX-87H column (Bio-Rad, USA) at 65°C. All compounds were detected with a refractive index detector, except for HMF and furfural, which were detected with a UV-detector (210nm).

To compensate for evaporated ethanol during the fermentations, the mole fraction of ethanol in the gas phase was assumed to be proportional to the mole fraction of ethanol in the liquid phase. The amount of evaporated ethanol could thereby be estimated by integration of the gas flow leaving the reaction multiplied with the mole fraction of ethanol in the gas, as described by Nilsson et.al., (18).

### Enzyme activities

### Preparation of cell extracts

Cell extracts were prepared for measurements of enzyme activities in the strains TMB3000 and CBS 8066. Crude extracts were made using Y-PER reagent (Pierce, Rockford, IL, USA). The cell extracts were kept in an ultra freezer (-80°C) until used. The protein content in the cell free preparation was determined by Coomassie Protein Assay Reagent using bovine serum albumin as a standard (Pierce, Rockford, IL, USA).

### Measurement of furfural and HMF reduction activity

Furfural and HMF reducing activity was measured according to Wahlbom et. al. (27). 20 µl of the cell free extract (diluted ten times in 100mM phosphate buffer) was diluted in 2.0 ml of 100 mM phosphate buffer (50 mM KH₂PO₄ and 50 mM K₂HPO₄) and furfural was added to a concentration of 10 mM. The samples were heated to 30°C and thereafter the reaction was started by addition of NADH to a concentration of 100 µM. The oxidation of NADH was followed as the change in absorbance at 340 nm. The same procedure was repeated with NADPH as the co-factor, but the sample amount was increased to 200 µl due to the lower activity. The total volume was still 2.0 ml and the concentrations of furfural and NADH 10 mM and 100 µM respectively.

The same procedure was repeated for measurement of HMF reduction capacity. 200 µl of diluted cell extract was used except for the measurement for strain TMB3000 with NADH as the co-factor where 20 µl sample was used due to the higher activity. The concentration of HMF was 10mM. Activities were measured with both NADH and NADPH.

### Measurement of ADH activity

ADH activity was measured according to Bruinenberg et. al. (28). The cell free extract was diluted 10 times and 20 µl of this dilution was added to 2.0 ml of 100 mM phosphate buffer. Ethanol was added to give a concentration of 100 mM. After heating to 30° the reaction was started by addition of NAD+ to a concentration of 100 µM. The reduction of NAD+ was followed as the change in absorbance at 340 nm.

### Continuous cultures

To analyze the mRNA content in strain CBS 8066 and TMB 3000 continuous cultures were run. The synthetic media was according to (25), but 33% more concentrated and the glucose concentration was 20 g/l. The liquid volume in the reactor (Belach BR 0.5 bioreactor, Belach Bioteknik AB, Solna, Sweden) was 500 ml and after the glucose in the batch had been consumed the feed was started at a dilution rate of 0.1 h⁻¹. The reactor was sparged with 300 ml of nitrogen /min. pH was maintained at 5.0 with 0.75 M NaOH and the temperature at 30°C. The stirrer speed was set to 500 rpm. To investigate which genes were induced by HMF, cell samples for mRNA analysis were taken both after feeding the reactor with media without HMF and with media including 0.5 g HMF I⁻¹. To get a steady state in the reactor the samples were taken 5 resident times after start of feed or change in feed media.

### mRNA preparation

Samples from the reactor were spinned in ice at 3000 rpm for 1 min and thereafter frozen in liquid nitrogen and stored at -80C until mRNA was isolated from the samples. The mRNA was isolated using Fast RNA kit (Q-biogene, USA). The mRNA was then purified, cDNA synthesized, *in-vitro* transcribed and fragmented as described by Affymetrix. Hybridization, washing, staining and scanning of microarray-chips (Yeast Genome S98 Arrays) were made with a Affymetrix Gene Chip Oven 640, a Fluidics Station 400 and a GeneArray Scanner (Affymetrix).

### ExClone

Selected strains (over expressing *LAT1, ALD6, ADH5, ADH6, GDH3, OYE3, IDP3, ADH7, AAD15, ERG27, HMG1, LYS5, SPS19, SGE1)* from the ExClone collection (Resgen, Invitrogen Corporation (UK)) were grown in 300 ml shake flasks (with carbon dioxide traps) containing 100 ml SD-Ura omission media and 40 g/l glucose as described by the supplier. However, 80 µM of Cu²⁺ was added when the shake flasks were inoculated and a 100 mM phosphate buffer were used. Samples for enzyme activity measurements were taken after 16 hours of growth at 30°C and 150 rpm.

### Results

Batch and fed-batch fermentations were performed with four yeast strains. After an initial batch growth phase on synthetic media, 1.5 liters of hydrolyzate was added to the reactor. In the batch fermentations hydrolyzate was added with the maximal rate (approximately 2000 ml/h), whereas in the fed-batch experiments the feed-rate was controlled using a closed-loop control algorithm. In short, the feed-rate was changed in a step-wise manner, in which the step increase was proportional to the derivative of the measured carbon dioxide evolution rate from the previous step. Feed rate control was obtained by controlling the frequency of a peristaltic pump (Watson-Marlow Alitea AB, Sweden). (see Materials and Methods).

### Batch fermentation

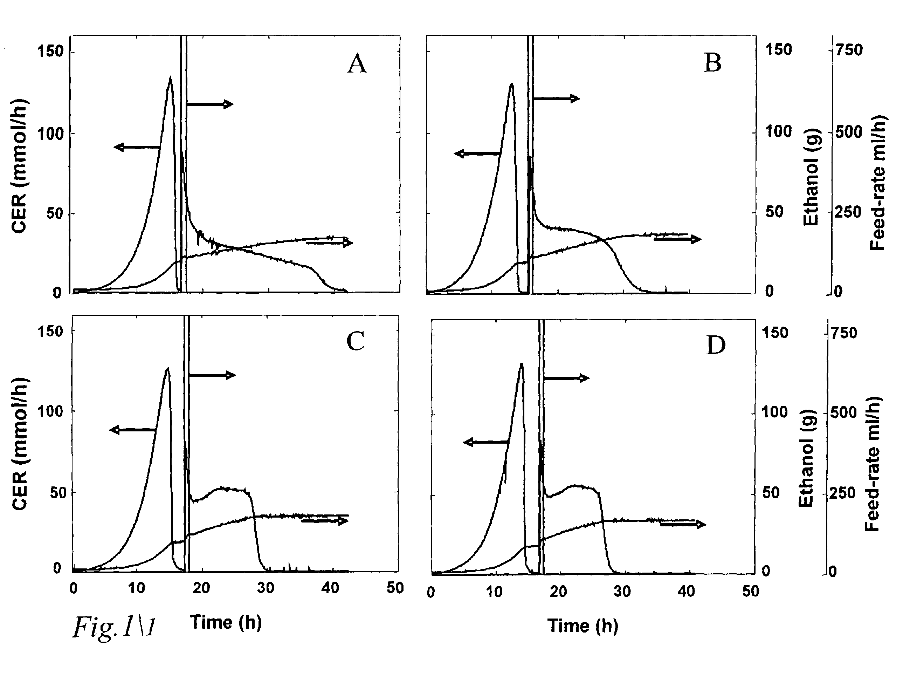

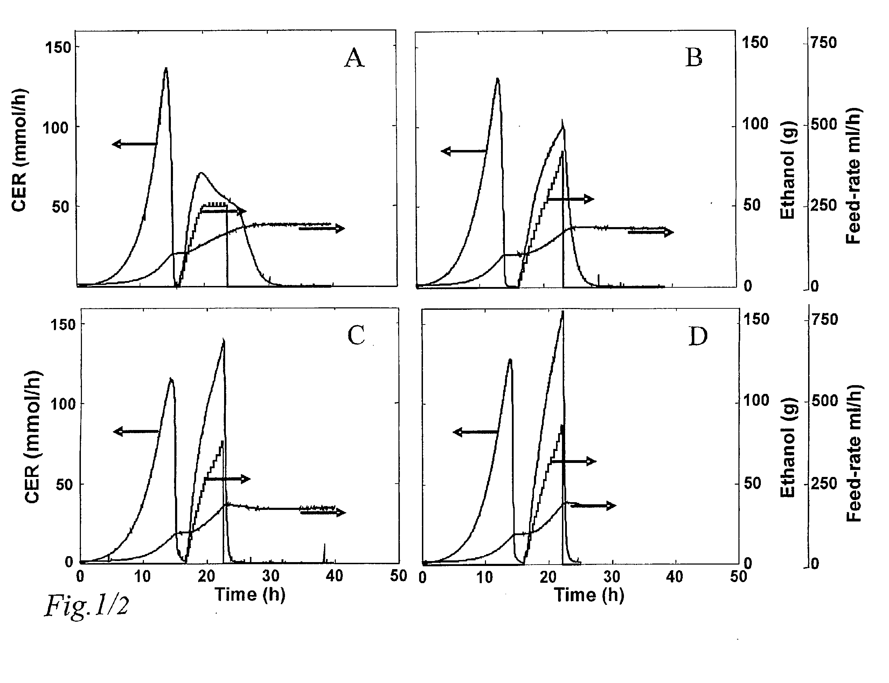

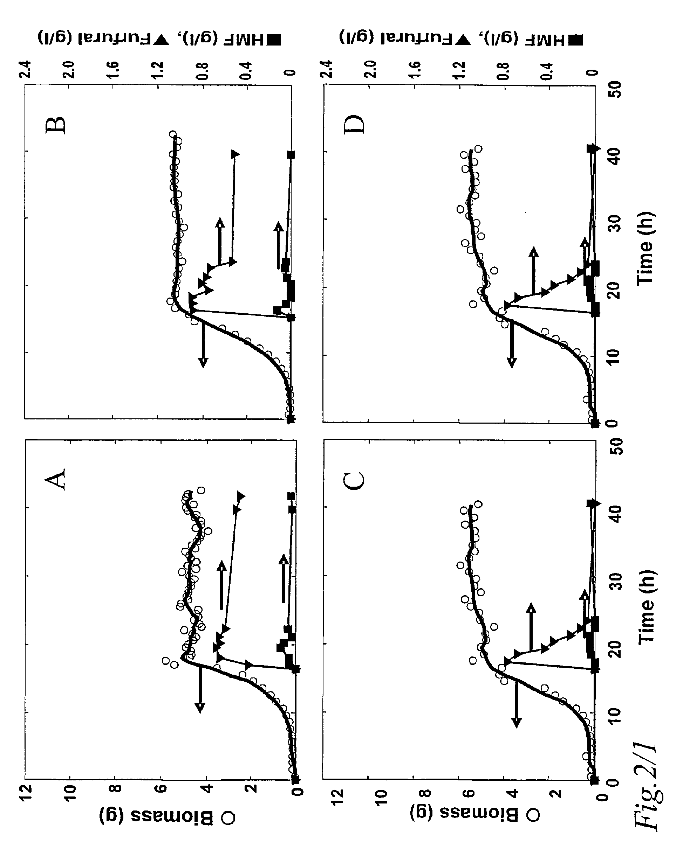

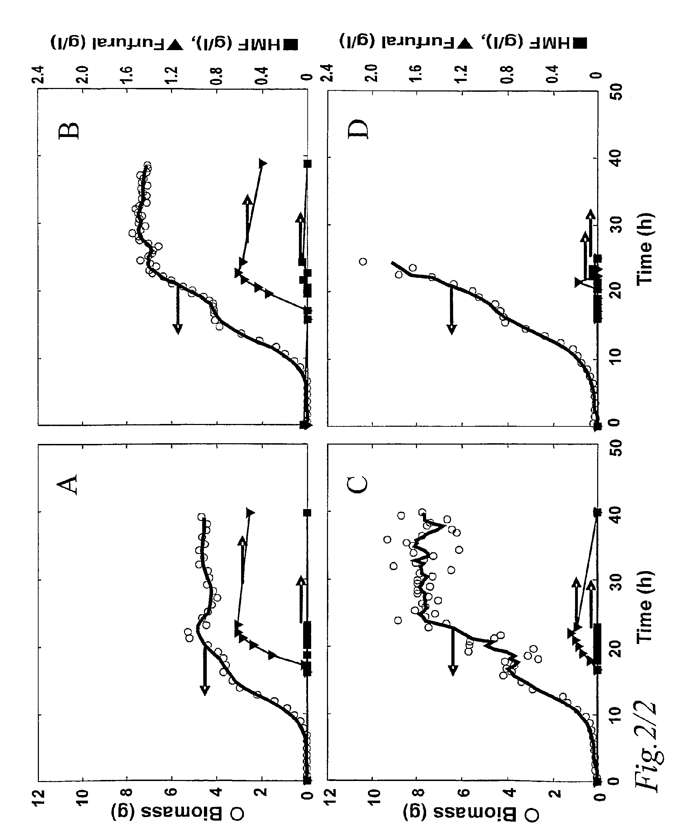

There were significant differences between the strains, in particular with respect to fermentation rates, as reflected by the carbon dioxide evolution rate (Fig 1). Also the specific growth rate, viability, and the conversion of the inhibitors HMF and furfural varied between the strains (Fig. 2). The specific ethanol productivity obtained with the strain CBS 8066 was clearly lower than for the other strains, and it gradually decreased throughout the fermentation for this strain, although the hexose sugars glucose and mannose were eventually completely consumed by all strains. None of the strains were able to grow in batch culture on hydrolyzate, but there were large differences with respect to maintenance of viability. The viability of strain CBS 8066 decreased to 16% within a few hours after the start of hydrolyzate fermentation (Table 2). In contrast, the strains with the highest average ethanol productivities, TMB3000 and TMB3006, had a viability of 77 and 100 %, respectively. These strains also had a more constant CER during the course of the fermentation, without the decrease seen for the other strains (Fig 1), and were able to decrease the concentrations of HMF to a greater extent than the other strains (Fig 2.). The productivity, viability and conversion of HMF the commercial baker's yeast was somewhere in between those of CBS 8066 and TMB3000.

### Fed-batch fermentation

The ethanol productivity was higher in fed-batch compared to batch fermentation for all strains tested (Table 3). For CBS 8066, the average ethanol productivity increased by 131 %. However, a gradual decrease in CER could not be avoided, and there was no cell growth, although a high viability was maintained. In fact, the viability was well maintained for all strains during fed-batch operation.

Apart from CBS 8066, the other strains grew in fed-batch fermentation (Fig. 2). The commercial baker's yeast strain had as high average ethanol productivity as CBS 8066 and for this strain CER also increased during the whole fed-batch phase. The average ethanol productivity was 100% higher for the most effectively fermenting strains, TMB3000 and TMB3006, than for CBS 8066. Importantly, these two strains were also able to grow in hydrolyzate with an "average" specific growth rate of around 0.12 h⁻¹. The concentrations of the furan inhibitors were maintained at very low levels (Fig. 2). The incorporation of the heterologous genes in TMB3006 apparently did not affect the inhibitor resistance or sugar flux rate, since TMB3006 behaved similar to TMB3000, in both batch and fed-batch fermentation.

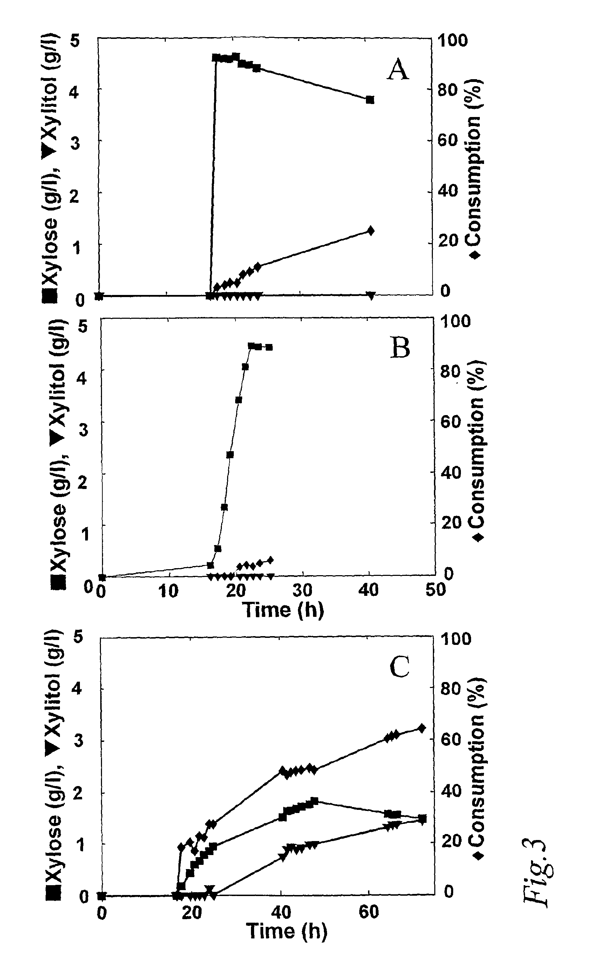

The strain carrying genes coding for XR, XDH and XK chromosomally integrated, TMB3006, consumed 6% of the xylose in the hydrolyzate. Xylose was assumed to be converted to ethanol, since no xylitol was detected (Fig. 3). To enhance xylose uptake in the fermentor the concentration of glucose and mannose was lowered by using fed-batch fermentations with low constant feed-rate. The feed-rate was set to 25 ml/h 17 hours after the start of the experiment and after 31 hours the feed-rate was decreased to 12.5 ml/h (Fig. 3). The xylose consumption for TMB3006 increased to 62%. However, 55% of the xylose consumed by TMB3006 was converted to xylitol.

### Enzyme activity

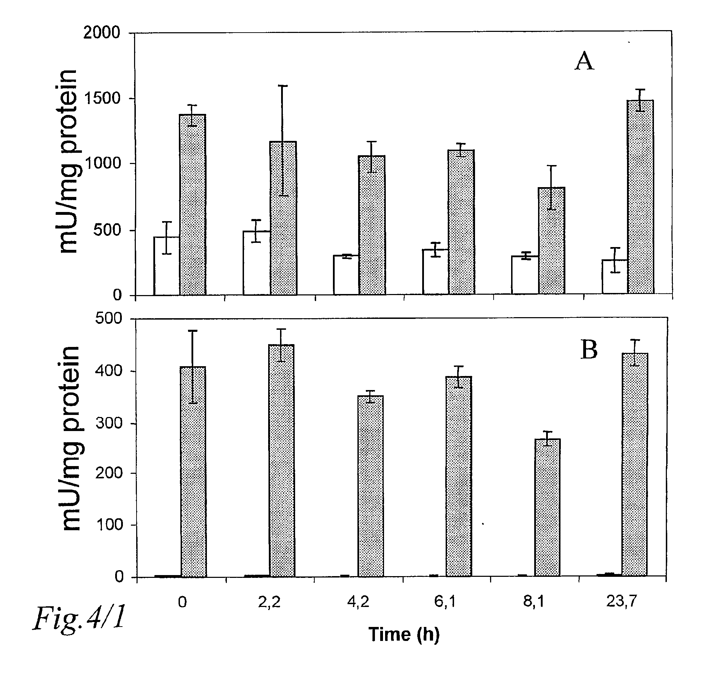

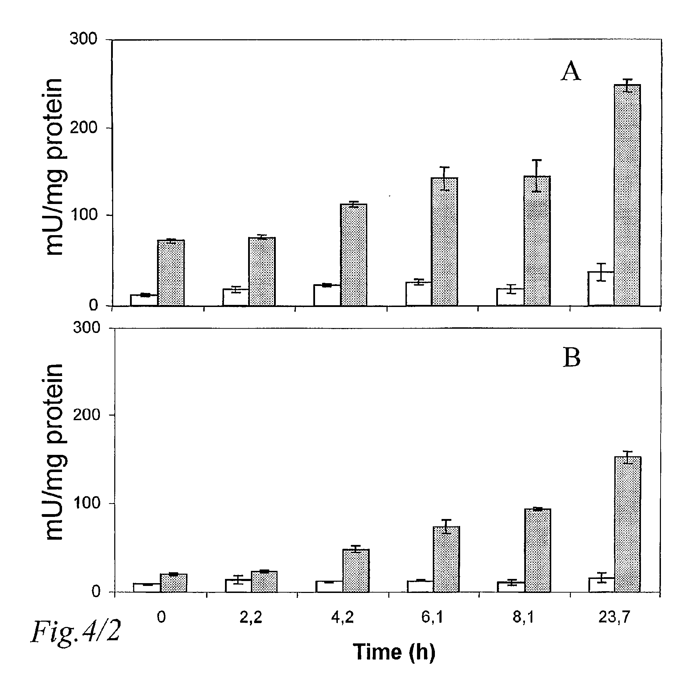

Furfural and HMF reduction capacity was measured on cell extract sampled during fed-batch experiments. The enzyme activities were measured with both NADH and NADPH as co-factors (Fig. 4). The activities were higher for TMB3000 than for CBS 8066 in all cases. For furfural reduction with NADH as the co-factor the activity for TMB3000 was twice as high as that of CBS 8066, and with NADPH as the co-factor it was 4 times as high. The largest difference was seen for HMF reduction activity using NADH as co-factor. This activity was very low in CBS 8066, but several hundredfold higher in TMB3000. Also with NADPH as co-factor it was higher, but only about 4 times higher.

Already before addition of any hydrolyzate, there was a clear difference between the activities in the two strains (Fig. 4). The NADH-dependent activity was almost constant during the fed-batch, indicating that the responsible enzyme(s) was probably not further induced by the hydrolyzate in any of the strains. With respect to the NADPH-dependent conversion, the strains behaved differently. The activity increased with time for TMB3000 but was almost constant for CBS 8066.

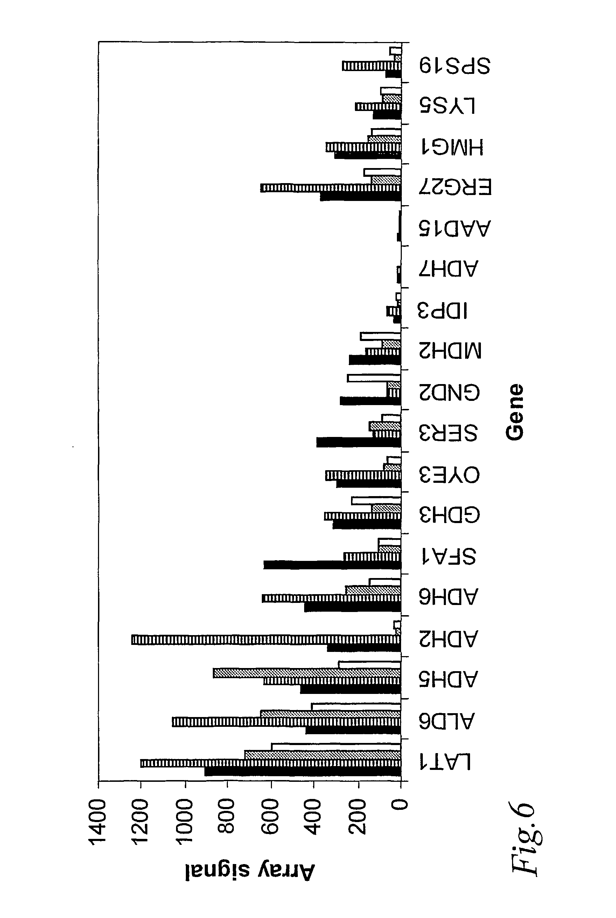

For TMB3000 the ADH activity was on average 40% higher than for CBS 8066 (Fig. 6). The difference in furfural conversion can thus not be explained by the mere difference in total ADH activity, but may be related to differences in the relative activity of different forms of ADH or strain specific changes in the ADH protein.

### Expression analysis

To investigate which enzyme(s) was responsible for the high conversion rates of in particular HMF and, in particular, with NADH as the cofactor, continuous cultivations where run with TMB 3000 and CBS 8066 with and without HMF present in the synthetic media. We searched for known reductase and hydrogenase genes that were upregulated at least twice in TMB 3000 in comparison with the strain CBS8066, both with or without the presence of HMF. -Maybe you should include the list here again- As seen in Fig 6, especially *SPS19* and *ADH2* turned out as promising good candidates since these where highly overexpressed in TMB 3000, and furthermore also induced by HMF.

### Test of mutants overexpressing selected target genes

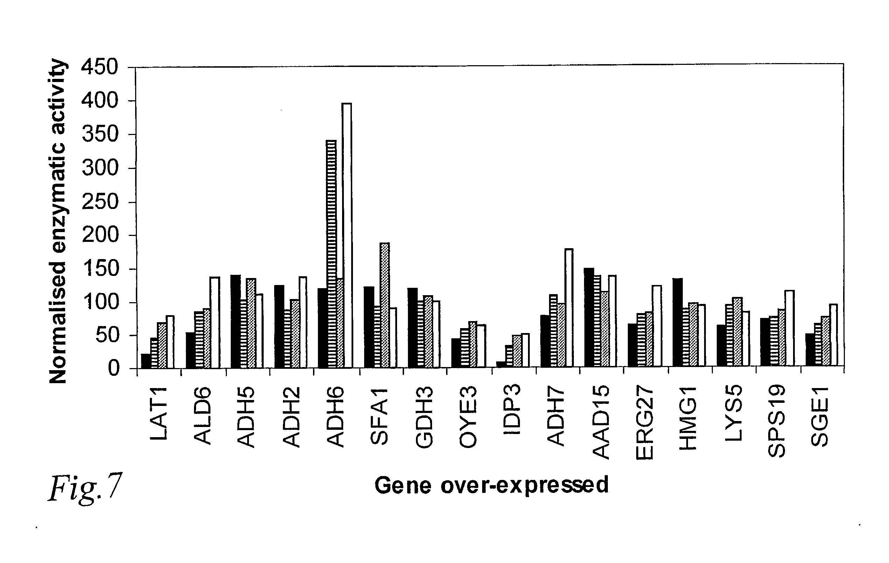

Strains from the ExClone collection in which the genes identified from the mRNA analysis described above were upregulated, were grown in shake flasks cultivations, and the obtained activities for reduction of furans were measured (Fig.7). Neither the strain over expressing *SPS19* or *ADH2* showed an increased ability to reduce HMF or furfural. However, strains over- expressing *SFA1, ADH6* and *ADH7* did have had an increased conversion ability. The activity found in the strain with *ADH6* upregulated was particularly pronounced. However, the co-factor preference was NADPH for conversion of both furfural and HMF. One strain -the SFA1 overexpressing strain- showed an increased conversion ability of HMF with NADH as the co-factor (as seen in for TMB 3000 compared to CBS 8066).

The present experiments clearly demonstrate that the ability of *S. cerevisiae* to ferment dilute-acid hydrolyzates of cellulosic material is highly strain specific. Importantly, and in accordance with previous work on the strain CBS 8066 (29, 20, 19, 18), higher productivities were obtained in fed-batch operation for all strains tested. The specific ethanol productivities for the most inhibitor tolerant strains (TMB3000 and TMB3006) increased by 69% in comparison to batch operation. Growth in batch cultivation was negligible for all strains, but the specific ethanol productivity varied significantly between strains also in batch fermentation. In contrast, all strains - with the exception of the strain CBS 8066 - to some extent grew in anaerobic fed-batch cultivations. The lower degree of inhibition in fed-batch cultivation is most likely attributed to the *in-situ* conversion of one or more inhibitors - including furan compounds and other aldehydes (30, 31, 22).

The physiological effects of furfural on *S. cerevisiae* have been previously studied extensively in synthetic model media. It has been shown in furfural-containing chemostat cultivation (both anaerobic and aerobic), that growth is inhibited if the specific furfural conversion rate exceeds a maximum critical conversion rate. During anaerobic conditions, the determining rate is the rate of reduction to furfuryl alcohol, whereas for aerobic conditions the critical rate is instead the oxidation rate to form furoic acid. At a too high furfural feed load, the furfural concentration increases in the medium, which presumably leads to inhibition of a number of key enzymes, including PDH and AIDH and washout occurs. For strain CBS 8066 the critical specific conversion rate of furfural was found to be between 0.10 and 0.15 g/g h during anaerobic conditions. In the present work, the concentration of furfural was very low (< 0.04 g/l) in all fed-batch cultivations, and it appears that the critical conversion rate of furfural was not exceeded. However, there were larger differences with respect to the HMF concentrations. In the cultivations with the best growing strains, TMB3000 and TMB3006, the HMF concentration was maintained at a relatively low level (< 0.23 g/l) whereas in the fed-batch fermentation with CBS 8066 only little conversion of HMF took place. For the two strains CBS 8066 and TMB3000, fed-batch fermentations were repeated and the activities of furfural and HMF reduction were measured (Fig. 4).

In an industrial medium the furfural concentration may 1 g/l up to 3 g/l depending on its origin.

Normally the furfuryl alcohol will be measured as the fermentation is anaerobic, and the product is then almost exclusively furfuryl alcohol.

The transformation capacity, conversion rate (determined by the enzymatic activity) determines how fast it is possible to add the furans. If they should be added too fast, furans will be accumulated in the medium, which will lead to an inhibition of central functions by means of interactions between furans and a number of enzymes such as PDH, PDC and others. This in turn leads to an inhibitor growth and down-regulation of the fermentation rate.

The average enzyme activities for furfural and HMF conversion in CBS 8066 was similar to those found for strain TMB3001, a strain derived from CEN.PK PK113-7A. The average activities for furfural conversion in CBS 8066 were 353 mU/mg protein with NADH as co-factor and 22.8 mU/mg protein with NADPH as co-factor, compared to 490 and 22 mU/mg protein, respectively, found in TMB3001. For HMF conversion, the average activities for CBS 8066 were 1.8 mU/mg protein (NADH) and 12.4 mU/mg protein (NADPH), compared with 2.2 and 22 mU/mg protein, respectively, for TMB3001. The Enzyme activities obtained for the strain TMB3000 were very different. The average furfural reduction activity was several times higher than for CBS 8066 and TMB3001, although the co-factor preference was similar. The most striking difference was, however, the high activity for HMF reduction with NADH as co-factor (Fig. 4). This activity was in fact more than 150 times higher for TMB3000 than for the other two strains. Also the NADPH coupled reduction rate was several times higher for TMB3000 than for CBS 8066.

The furfural and HMF conversion activities provide an explanation for the advantage of TMB3000 over CBS 8066 in lignocellulose fermentation. High activities ensure high conversion rates of furfural and HMF, and possibly other inhibitory aldehydes (32), so that the concentration of these inhibitors is kept low in the fermentation. For strain CBS 8066 the measured *in vitro* activity for furfural reduction would correspond to an *in vivo* reduction rate of 0.69 g/g h. This, in fact agrees well with the maximum conversion rate reported in synthetic media for the same strain (0.6 g/g h). The corresponding predicted specific conversion rate of HMF would be 0.03 g/g h, which is somewhat lower than the value reported in synthetic media of 0.14 g/g h. For strain TMB3000 measured *in vitro* reduction activities for furfural and HMF were 2.26 g/g h and 0.98 g/g h respectively and this would correspond to feeding rates of about 3 I/h, at the cell density and volumes used which is much higher than those applied in the present work (cf. Fig. 2). For CBS 8066, however, the activities for HMF conversion correspond to a feeding rate of only 100 ml/h. Another factor to consider is the difference in co-factor preference for HMF conversion. Since NADH is the preferred co-factor in the conversion of HMF, there will be no drain of NADPH competing with anabolic reactions in strain TMB3000.

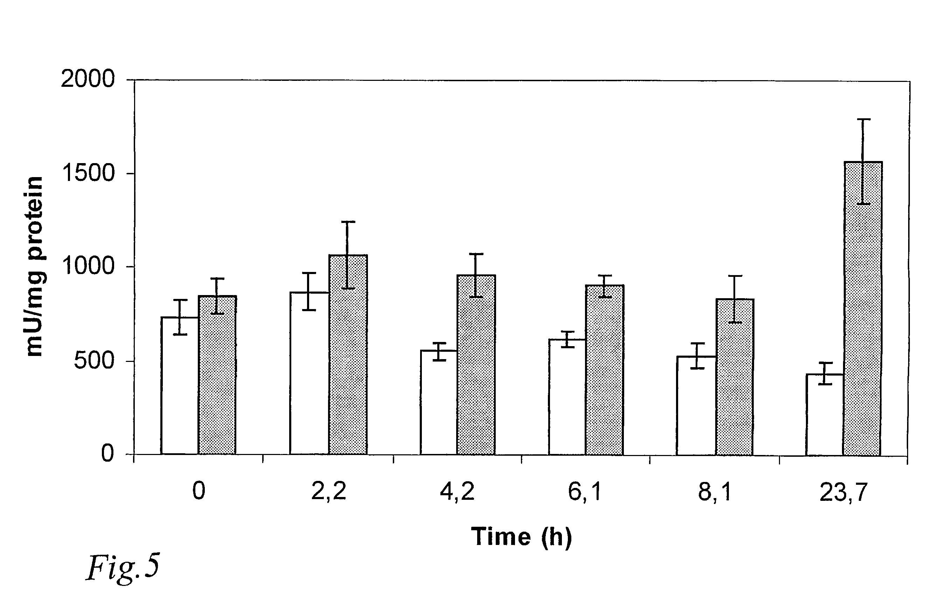

There was a considerable furan reduction activity in the cell extract already before the cells had been exposed to the inhibitors of the hydrolyzate, and furthermore, activity measurements showed that the furan reduction activity did not increase significantly with time during exposure to hydrolyzate, indicating that the responsible enzyme(s) were not induced. The ability to reduce furfural has previously been attributed to the enzyme alcohol dehydrogenase (ADH) (32, 33), although this has been questioned (35). The ratio between measured ADH activities for CBS 8066 and TMB3000 (Fig. 5) was much close to 1 than the than ratio between corresponding the furfural reduction activities. This finding suggests that also other enzymes may be important in the conversion of furfural, or that ADHs in different strains may have different affinities for furfural due to e.g. point mutations.

Below it is further shown that the enzyme encoded by the gene *ADH6* in *Saccharomyces cerevisiae* is able to convert HMF using the co-factor NADPH. Yeast strains that overexpress this gene have a substantially higher conversion rate of HMF in both aerobic and anaerobic cultures. Importantly, we have furthermore shown that strains over-expressing *ADH6* has a substantially higher ethanol productivity and are less effected by inhibition during fermentation of a dilute-acid lignocellulose hydrolyzate. Strains genetically modified to give a high expression of *ADH6* will therefore be advantageous for the conversion of lignocellulosic hydrolyzates.

### Materials and Methods

### Strains and genetic constructs

The alcohol dehydrogenase VI *(ADH6)* gene from *Saccharomyces cerevisiae* TMB3000 and CEN.PK 113-5D were amplified using the primers ADH6-FOR and ADH6-REV (Table 4). The 5' region of the primers ADH6-FOR and ADH6-REV contain 34 and 33 nucleotides corresponding to the sequence of the HXT promoter and PGK1 terminator, respectively. After PCR amplification, the PCR products were analyzed by electrophoresis in agarose gels and purified using QIAquick PCR Purification kit (QIAGEN). The vector pYEplacHXT was linearized using the restriction endonuclease *Bam* HI. A mix containing the linear vector (6.2 Kb), the ADH6 product from TMB 3000 (T-ADH6) or CEN.PK 113-5D (C-ADH6) was used to transform *S*. *cerevisiae* CEN.PK 113-5D by lithium acetate method (38). Yeast cells were grown overnight, in 5 mL YPD, at 30 °C. In the morning, a 50 mL YPD solution was inoculated using 3 mL of the pre-culture. Growth was followed until OD₆₀₀ = 1.2, when the yeast cells were centrifuged and finally suspended in 10 mL of sterile water. One milliliter of cells were pipetted in micro-centrifuge tubes and centrifuged for approximately 20 seconds in top speed. After supernatant removal, the cells were resuspended in 1 mL of 100 mM lithium acetate (LiAc) and incubated at 30 °C for 10-15 minutes. The suspension was centrifuged at top speed for 30 seconds, the supernatant removed and the transformation mix (240 µl PEG 50% w/v, 36 µl 1.0 M lithium acetate, 52 µl 2 mg/mL ssDNA, 28 µl sterile water, 1.0 µl 40 ng/µl pYEplacHXT vector and 3 µl 40 ng/µl PCR product) was added to the pellet. After subsequent incubations at 30 °C for 30 min and 42 °C for 20 min, the mix was centrifuged at top speed for 30 seconds and the transformation mix removed. The yeast cells were resuspended in 150 µl of YNB and left at room temperature for approximately 2 hours. After incubation the mix containing cells was plated on YNB-plates, which were incubated at 30 °C for 3-4 days. A yeast control strain was constructed by transformation with the empty pYEplacHXT vector. Transformant yeast strains were selected by colony PCR using ADH6 primers and ethanol oxidation capacity. Plasmids from two transformants (C-ADH6-2 and T-ADH6-2) were recovered, amplified in *E. coli* DH5α and submitted to automatic sequencing.

### Cultivation conditions

### Shake flasks

Growth experiments were carried out in 300 ml unbaffled shake-flasks. The volume of synthetic media was 200 ml with the composition given in (25) and contained 13 g glucose. The pH was adjusted to 5.5 with 2 M NaOH at the start of the cultivations. The shaker speed was 170 rpm and the temperature was 30°C. The anaerobic shake flasks were equipped with glycerol traps, whereas the aerobic shake flasks were sparged with air. When OD₆₂₀ reached 3.0 the pH was readjusted to 5.5 and HMF was added to a concentration of 1.5 g/l.

### Bioreactor experiments

Batch fermentations were made with the strain CEN.PK 113-5D and T/ADH6-2. The reactor (Belach BR 0.5 bioreactor, Belach Bioteknik AB, Solna, Sweden) was initially filled with 300 ml synthetic media according to (25), which contained 30 g glucose. pH was maintained at 5.0 with 0.75 M NaOH and the temperature at 30°C. The reactor was sparged with 300 ml/min of nitrogen and the stirrer speed was set to 500 rpm. When the carbon dioxide evolution rate had reached a maximum, 300 ml hydrolyzate was added.

The hydrolyzate used was produced from forest residue, originating mainly from spruce, in a two-stage dilute-acid hydrolysis process using sulphuric acid as the catalyst (19). The hydrolyzates obtained from the two stages were mixed and stored at 8°C until used. The composition of the hydrolyzate is given in Table 5.

### Measurement of enzyme activity

Cell extracts of strains over-expressing *ADH6* were prepared for measurements of enzyme activities. Crude extracts were made using Y-PER reagent (Pierce, Rockford, IL, USA). The protein content in the cell free preparation was determined using Micro BCA Protein Assay Kit (Pierce).

Enzyme activities for theoxidation of ethanol and the reduction of furfural, 5-hydroxymethyl-furfural (HMF) and dihydroxyacetone phosphate (DHAP) were measured on cell extract samples. The rate of ethanol oxidation was determined by monitoring the reduction of NAD⁺ photometrically at a wavelength of 340 nm. The enzyme assay, based on (37), contained 5.0 mM NAD⁺ and 1.7 M of ethanol in 100 mM glycine buffer at pH 9.0 in 1.0 cm path length cuvettes. The samples were incubated at 30 °C and the reaction was started by addition of ethanol. HMF and furfural reducing activities were measured according to (27). 5-10 µL of cell free extract (using different dilutions) was diluted in 1 mL of 100 mM phosphate buffer (50 mM KH₂PO₄ and 50 mM K₂HPO₄) and NADH was added to a concentration of 100 µM. The samples were incubated at 30 °C and thereafter the reaction was started by addition of HMF or furfural to a concentration of 10 mM. The oxidation of NADPH was followed as the change in absorbance at 340 nm. The procedure was repeated with NADH as the co-factor, but the sample amount was increased due to the lower activity. The total volume was still 1.0 mL. The same procedure was carried out when using DHAP, except that only 0.7 mM of this substrate was used. The molar absorption coefficient (s) used for NADH and NADPH was ε₃₄₀ = 6.22 mM⁻¹cm⁻¹.

### Results

### Selection of transformants

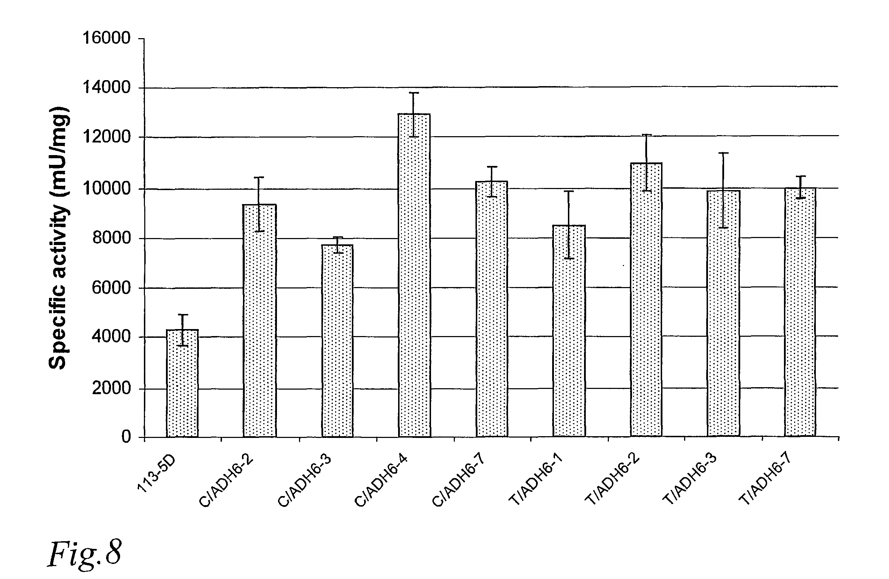

*ADH6* gene was PCR amplified from CEN.PK or TMB3000 genomic DNA and cloned into the yeast vector pYEplac-HXT, generating pYEplacHXT-C/ADH6 and pYEplacHXT-T/ADH6 vectors respectively. The plasmids were used for the transformation of CEN.PK113-5D strain. Colony PCR was used to select yeast strains that carried a pYEplacHXT-ADH6 vector. Clones having the *ADH6* gene from CEN.PK and TMB3000 were called C/ADH6-m (m=1, 2 etc) and T/ADH6-n (n=1, 2, etc), respectively). Clones with increased expression of *ADH6* gene were selected amongst transformants for their increased ethanol oxidation capacity compared to the control strain CEN.PK113-5D carrying the empty vector YEplac-HXT (Fig. 8).

### In vitro reduction capacity

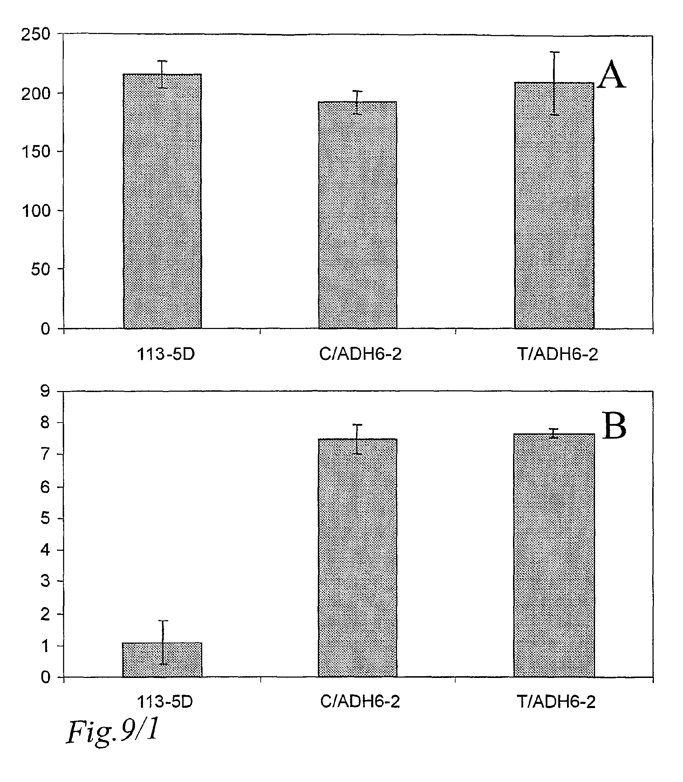

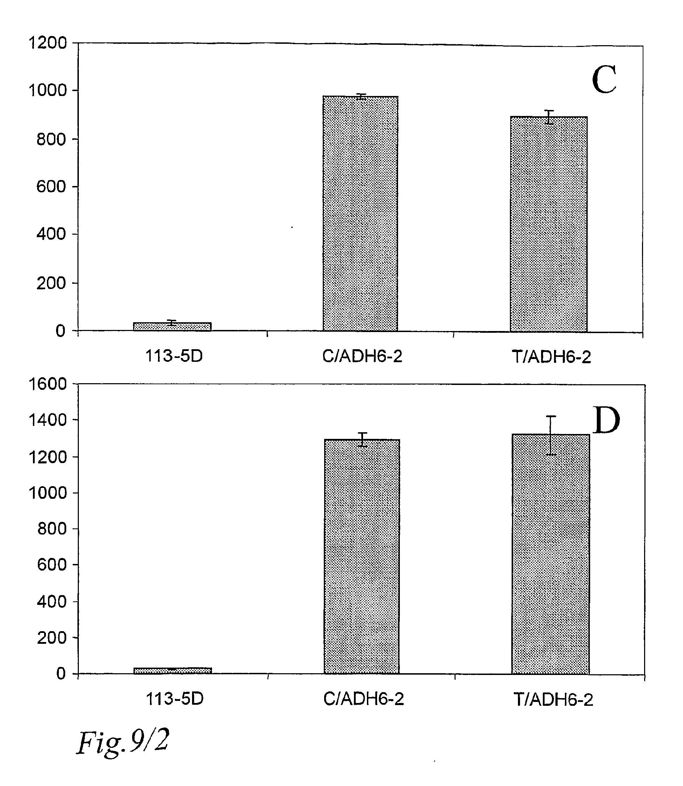

HMF and furfural conversion capacity of *ADH6* over-expressing strains was analyzed using NADH and NADPH as cofactors in enzymatic assays (Fig. 9). Strains overexpressing *ADH6* were able to convert HMF using NADPH as well as NADH as cofactor, but the activity using NADH was clearly lower than with NADPH. Moreover, similar values for HMF and furfural conversion were obtained for C- and T-ADH6 strains, which suggest no differences in protein structures/activity between CEN.PK and TMB3000 strains. Indeed, the *ADH6* gene sequences from the two strains did not show any significant difference, except for a substitution of the G-203 in C-ADH6-2 for E-203 in T-ADH6-2. When compared with the control strain CEN.PK113-5D (pYEplac-HXT), cell extracts from ADH6 over-expressing strains show approximately 9 fold higher NADH-dependent HMF activity, whereas *ADH6*-dependent HMF reduction was increased more than 100 times when using NADPH as cofactor (Fig. 9). These results confirm previous reports that propose *ADH6* as NADPH-dependent enzyme for reduction of other compounds (38). Furfural reduction was possible only when using NADPH as cofactor (Fig. 9).

### In vivo reduction capacity

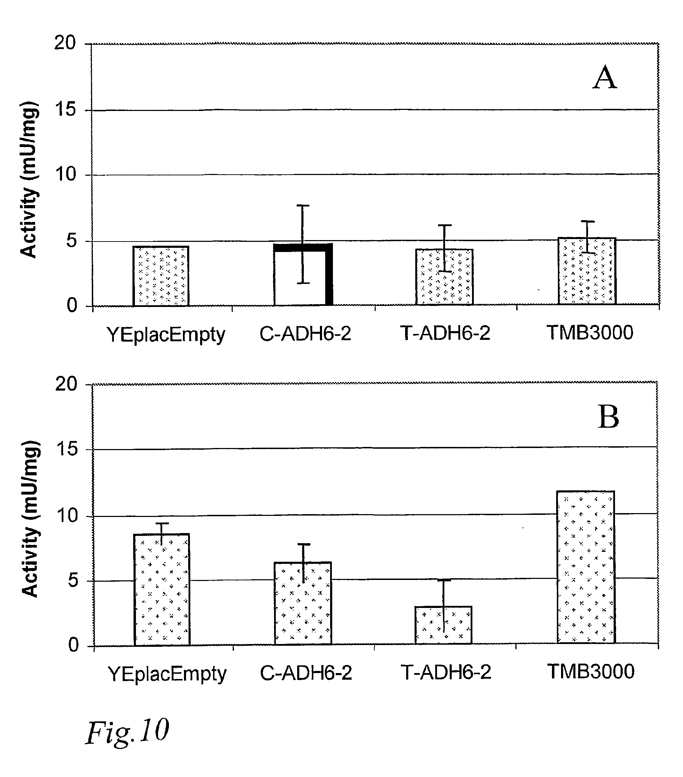

*In vivo* HMF conversion was analyzed in minimal medium using aerobic and anaerobic conditions in shake-flasks. The *ADH6* over-expressing strains showed higher specific HMF uptake (3.5-3.9 fold) in aerobic as well as in anaerobic conditions (Tables 6 and 7). The specific uptake of HMF appeared correlated with an increase in glycerol production (Tables 6 and 7). In order to analyze a possible direct activity of *ADH6* gene product in the glycerol metabolic pathway, the C-ADH6-2 and T-ADH6-2 DHAP reduction capacity was analyzed by enzymatic assays. Enzyme activity measurements did not shown any increase in DHAP reduction (Fig. 10). Possibly, the higher glycerol production is indirectly related to the HMF reduction via cellular co-factor balances.

### Tolerance to dilute-acid hydrolyzate

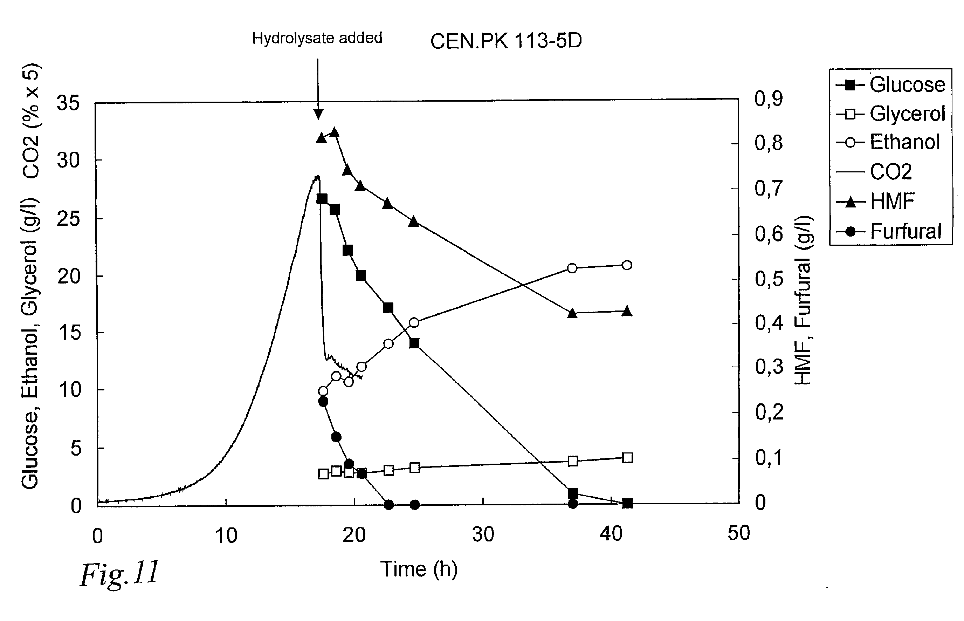

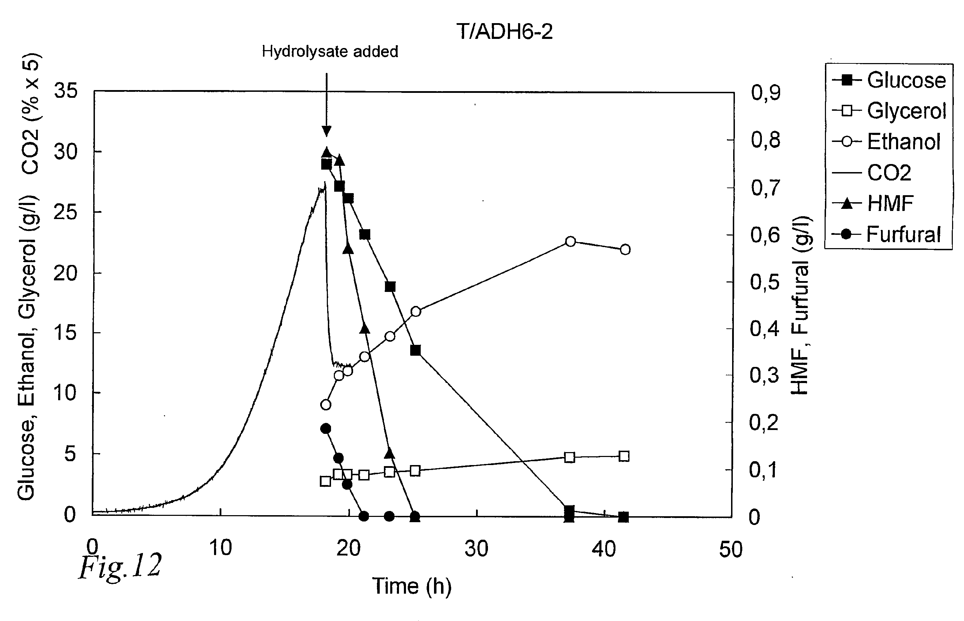

The control strain and a strain over-expressing *ADH6* from TMB3000 (T/ADH6-2) were used in anaerobic batch fermentations with a dilute-acid hydrolyzate (Fig. 11 and 12). T/ADH6-2 strain was clearly less inhibited than the control strain and the CER did not decrease as rapidly for T/ADH6-2 as for the control strain (Fig. 11 and 12). This is also reflected in the specific ethanol productivity, which was 35% higher for T/ADH6-2 compared to the control strain. The specific uptake rate of HMF was found to be five-fold higher in the T/ADH6-2 than in the control strain (0.05 g/g h and 0.01 g/g h, for T/AHD6-2 and the control strain, respectively). The specific uptake rate of furfural was, however, the same (0.02 g/g h) for both strains, showing that the tolerance is not linked to the furfural, but to the HMF conversion capacity.

Conclusions drawn from this latter experiment series are:
1. Strains over-expressing *ADH6* gene show higher HMF conversion rate under aerobic as well as in anaerobic conditions, in both synthetic media and dilute-acid hydrolyzates.
2. HMF conversion by *ADH6* gene product is mostly NADPH dependent, since *in vitro* enzyme activity assays using this cofactor show 100 times more activity than that with NADH.
3. A strain overexpressing *ADH6* had a 35% higher fermentation rate of undetoxified dilute-acid hydrolyzate than the corresponding control strain.

**Table 1. Description of the five different strains of S. cerevisae used in this work.**

| **Strain** | **Description** | **Reference** |
|---|---|---|
| CBS 8066 | A widely used diploid laboratory strain | (21) |
| Baker's yeast | Commercially available yeast obtained from the Swedish - Baker's yeast company, Jästbolaget AB, Rotebro, Sweden | - |
| TMB3000 | A strain isolated from a spent sulfite liquor fermentation plant | (32) |
| TMB3006 | A genetically modified strain based on TMB3000. Expresses the heterologous genes *XYL1* and *XYL2* from *P. stipitis* and overexpresses the gene *XKS1* from *S. cerevisiae.* | (17) |

**Table 2. Composition of hydrolyzate. The hydrolyzate used as a substrate in the fermentations was produced from forest residue, originating mainly from spruce, in a two-stage dilute-acid hydrolysis process using sulphuric acid. The hydrolysis was performed as reported in Nilsson et.al.**

| Compound | Concentration (g/l) |
|---|---|
| Glucose | 16.2 |
| Mannose | 13.4 |
| Galactose | 3.2 |
| Xylose | 6.1 |
| Arabinose | 1.1 |
| Acetic acid | 1.5 |
| Furfural | 0.2 |
| HMF | 1.6 |

**Table 3. Average ethanol productivity, CFU and specific growth rates obtained in batch and fed-batch cultivations using dilute-acid hydrolyzate as carbon source.**

| | Mode of cultivation | CBS 8066 | Baker's yeast | TMB3000 | TMB3006 |
|---|---|---|---|---|---|
| Specific ethanol | Batch | 0.13 | 0.19 | 0.36 | 0.30 |
| productivity, rₑ (g/g h) | Fed-batch | 0.30 | 0.31 | 0.61 | 0.66 |
| CFU (%)² | Batch | 16 | 4 | 77 | 100 |
| | Fed-batch | 78 | 100 | 95 | 81 |
| Average specific | Batch | 0 | 0 | 0 | <0.01 |
| growth rate, µ (h⁻¹) | Fed-batch | 0 | 0.07 | 0.12 | 0.12 |

| | | | | | |
|---|---|---|---|---|---|
| ¹Calculated as the average specific ethanol productivity during the fermentation of hydrolyzate, until CER decreased to less than 5 mmol/h. ²CFU value taken a 2-8 hours after start of the feeding of hydrolyzate (%) | | | | | |

**Table 4 - Primers for ADH6 amplification.**

| **Primer** | **Sequence (5' to 3')** | **Size** |
|---|---|---|
| DH6-FOR | TTAATTTTAATCAAAAAGGATCCCCGGGCTGCAATGTCTTATCCTGAG AAATTTGAAGG | **0 bp** |
| DH6-REV | CACCACCAGTAGAGACATGGGAGATCTAGAATTCCTAGTCTGAAAATTC TTTGTCGTAGC | **0 bp** |

| | | |
|---|---|---|
| • Upper-case letters: homolog sequences for HXT promoter in ADH6-FOR primer and PGK terminator in ADH6-REV, respectively. | | |

**Table 5 - Composition of hydrolyzate. The hydrolyzate was produced in a two-stage dilute-acid hydrolysis of forest residues, mainly from spruce.**

| Compound | | Concentration |
|---|---|---|
| | (g/l) | |
| Glucose | | 23.7 |
| Mannose | | 13.6 |
| Galactose | | 3.0 |
| Xylose | | 5.2 |
| HMF | | 2.0 |
| Furfural | | 0.6 |
| Acetic acid | | 1.6 |

**Table 6 - Anaerobic cultivations of ADH6 clones**

| Strain | Specific growth rate (h⁻¹) without HMF | Specific growth rate (h⁻¹) with 1.5 g/l HMF | Specific uptake of HMF (g/gh) | Glycerol yield (g/g) | Biomass yield (g/g) |
|---|---|---|---|---|---|
| CEN.PK 113 | 0,38 | 0,21 | 0,12 | 0,072 | 0,059 |
| TMB3000 | 0,45 | 0,25 | 0,31 | 0,086 | 0.074 |
| C/ADH6-2 | 0,34 | 0,21 | 0,42 | 0,101 | 0,064 |
| T/ADH6-2 | 0,34 2 | 0,21 | 0,44 | 0,097 | 0,055 |

**Table 7 - Aerobic cultivations of ADH6 clones**

| Strain | Specific growth rate (h⁻¹) without HMF | Specific growth rate (h⁻¹) with 1.5 g/l HMF | Specific uptake of HMF (g/gh) | Glycerol yield (g/g) | Biomass yield (g/g) |
|---|---|---|---|---|---|
| CEN.PK 113 | 0,43 | 0,29 | 0,20 | 0,049 | 0,099 |
| TMB3000 | 0,44 | 0,33 | 0,29 | 0,057 | 0,092 |
| C/ADH6-2 | 0,35 | 0,32 | 0,78 | 0,085 | 0,077 |
| T/ADH6-2 | 0,37 | 0,33 | 0,80 | 0,083 | 0,078 |

### REFERENCES

1. Bergeron, P. W., Wright, J. D. and Wyman, C. E., Dilute acid hydrolysis of biomass for ethanol production. 1989, 12 1277-1296
2. Galbe, M. and Zacchi, G., A review of the production of ethanol from softwood. 2002, 59 618-628
3. Larsson, S., Palmqvist, E., Hahn-Hägerdal, B., Tengborg, C., Stenberg, K., Zacchi, G. and Nilvebrant, N., The generation of fermentation inhibitors during dilute acid hydrolysis of softwood. 1999, 24 151-159
4. Larsson, S., Quintana-Sáinz, A., Reimann, A., Nilvebrant, N. O. and Jönsson, L., Influence of lignocellulose-derived aromatic compounds on oxygene-limited growth and ethanol fermentation by Saccharomyces cerevisiae. 2000, 84-86 617-631
5. Palmqvist, E., Almeida, J. and Hahn-Hägerdal, B., Influence of furfural on anaerobic glycolytic kinetics of Saccharomyces cerevisiae in batch culture. 1999, 62 447-457
6. Taherzadeh, M., Eklund, R., Gustafsson, L., Niklasson, C. and Lidén, G., Characterization and fermentation of dilute-acid hydrolyzates from wood. 1997, 36 4659-4665
7. Taherzadeh, M. J., Gustafsson, L., Niklasson, C. and Liden, G., Physiological effects of 5-hydroxymethylfurfural on Saccharomyces cerevisiae. 2000, 53 701-708
8. Olsson, L. and Hahn-Hägerdal, B., Fermentation of lignocellulosic hydrolysates for ethanol production. 1996, 18 312-331
9. Taherzadeh, M., Gustafsson, L., Niklasson, C. and Lidén, G., Conversion of furfural in aerobic and anaerobic batch fermentation of glucose by Saccharomyces cerevisiae. 1999, 87 169-174
10. Villa, G., Bartroli, R., Lopez, R., Guerra, M., Enrique, M., Penas, M., Rodriguez, E., Redondo, D., Iglesias, I. and Diaz, M., Microbial transformation of furfural to furfuryl alcohol by Saccharomyces cerevisiae. 1992, 12 509-512
11. Skoog, K. and Hahn-Hägerdal, B., Effect of oxygenation on xylose fermentation by Pichia stipitis. 1990, 56 3389-3394
12. Ho, N. W. and Chang, S., Cloning of yeast xylulokinase gene by complementation of Escherichia coli and yeast mutations. 1989, 11 417-421
13. Jeppsson, H., Yu, S. and Hahn-Hägerdal, B., Xylulose and glucose fermentation by Saccharomyces cerevisiae in chemostat culture. 1996, 62 1705-1709
14. Ho, N., Chen, Z. and Brainard, A., Genetically engineered Saccharomyces yeast capable of effective cofermentation of glucose and xylose. 1998, 64 1852-1859
15. Johansson, B., Christensson, C., Hobley, T. and Hahn-Hägerdal, B., Xylulokinase overexpression in two strains of Saccharomyces cerevisiae also expressing xylose reductase and xylitol dehydrogenase and its effect on fermentation of xylose and lignicellulosic hydrolysate. 2001, 67 4249-4255
16. Toivari, M., Aristidou, A., Ruohonen, L. and Pentillä, M., Conversion of xylose to ethanol by recombinant Saccharomyces cerevisiae: Importance of xylulokinase (XKS1) and oxygen availability. 2001, 3 236-249
17. Johansson, B., Metabolic engineering of the pentose phosphate pathway of Xylose fermenting Saccharomyces cerevisiae. 2001,
18. Nilsson, A., Taherzadeh, M. and Lidén, G., On-line estimation of sugar concentration for control of fed-batch fermentation of lignocellulosic hydrolyzates by Saccharomyces cerevisiae. 2002, 25 183-191
19. Nilsson, A., Taherzadeh, M. J. and Liden, G., Use of dynamic step response for control of fed-batch conversion of lignocellulosic hydrolyzates to ethanol. 2001, 89 41-53
20. +Taherzadeh, M. J., Niklasson, C. and Liden, G., On-line control of fed-batch fermentation of dilute-acid hydrolyzates. 2000, 69 330-338
21. Verduyn, C., Postma, E., Scheffers, W. A. and van Dijken, J. P., Physiology of Saccharomyces cerevisiae in anaerobic glucose-limited chemostat cultures. 1990, 136 395-403
22. Martín, C. and Jönsson, L., Comparison of resistance of industrial and laboratory strains of Saccharomyces cerevisiae and Zygosaccharomyces to lignocellulose-derived fermentation inhibitors. 2003, 32 386-395
23. Busturia, A. and Lagunas, R., Catabolic inactivation of the glucose transport system in Saccharomyces cerevisiae. 1986, 132 379-385
24. Meinander, N., Boels, I. and Hahn-Hägerdal, B., Fermentation of xylose/glucose mixtures by metabolically engineered Saccharomyces cerevisiae strains expressing XYL1 and XYL2 from Pichia stipitis with and without overexpressing TAL1. 1999, 68 79-87
25. Taherzadeh, M., Lidén, G., Gustafsson, L. and Nikiasson, C., The effects of pantothenate deficiency and acetate addition on anaerobic batch fermentation of glucose by Saccharomyces cerevisiae. 1996, 46 176-182
26. Björkqvist, S., Ansell, R., Alder, R. and Lidén, G., Glycerol-3-phosphate dehydrogenase mutants of Saccharomyces cerevisiae grown under aerobic and anaerobic conditions. 1997, 63 128-132
27. Wahlbom, C. and Hahn-Hägerdal, B., Furfural, 5-hydroxymethyl furfural and acetoin act as external electron acceptors during anaerobic fermentation of xylose by recombinant Saccharomyces cerevisiae. 2002, 78 172-178
28. Bruinenberg, P., Van Dijken, J. and Scheffers, W., An enzymatic analysis of NADPH production and consumption in Candida utilis. 1983, 129 965-971
29. Taherzadeh, M., Niklasson, C. and Liden, G., Conversion of dilute-acid hydrolyzates of spruce and birch to ethanol by fed-batch fermentation. 1999, 69 59-66
30. Chung, I. S. and Lee, Y. Y., Ethanol fermentation of crude acid hydrolyzate of cellulose using high-level yeast inocula. 1985, 27 308-315
31. Nemirovskii, V. and Kostenko, V., Transformation of yeast growth inhibitors, whish occurs during biochemical processing of wood hydrolyzates. 1991, 1 16-17
32. Lindén, T., Peetre, J. and Hahn-Hägerdal, B., Isolation and caracterisation of acetic acid-tolerant galactose-fermenting strain of S. cerevisiae from a spent sulphite liquor fermentation plant. 1992, 15 103-121
33. Diaz, D., Villa, P., Guerra, M., Rodriguez, E., Redondo, D. and Martinez, A., Conversion of furfural into furfuryl alcohol by Saccharomyces cerevisiae 354. 1992, 12 351-354
34. Modig, T., Lidén, G. and Taherzadeh, M., Inhibition effects of furfural on alcohol dehydrogenase, aldehyde dehydrogenase and pyruvate dehydrogenase. 2002, 363 769-776
35. Larsson, S., Fermentation inhibitors, detoxification and genetic engineering of Saccharomyces cerevisiae for enhanced resistance. 2000,
36. Lagunas, R., Dominguez, C., Busturia, A. and Saez, M., Mechanisms of appearance of the pasteur effect in Saccharomyces cerevisiae: inactivation of sugar transport systems. 1982, Oct 19-25
37. Methods in Enzymatic Analysis 1974. HU Bergmeyer (Ed.) Vol 1, p. 428-429.
38. Giertz, R. D., and R. A. Woods. 1998. in "Methods in Microbiology". Vol. 26 (AJP Brown and MF Tuite, eds) p.53. Academic Press, San Diego.
39. Larroy, C., M. R. Fernandez, G. E, X. Pares, and J. A. Biosca. 2002. Characterization of the Saccharomyces cerevisiae YMR318C (ADH6) gene product as a broad specifity NADPH-dependant alcohol dehydrogenase: relevance in aldehyde reduction. Biochem J. 361:163-172.

### Figure captions

Fig.1. After an initial batch on synthetic media, 1.5 liters of hydrolyzate was added to the reactor. *Top row:* Batch fermentations where hydrolyzate was added with maximal rate (approximately 2000 ml/h). *Bottom row:* Fed-batch where the feed-rate was controlled by the program previously developed (see Materials and Methods). Both batch and fed-batch fermentations was performed with *A:* CBS 8066, *B:* Baker's yeast, *C:* TMB3000, *D:* TMB3006. *Left scale:* carbon evolution rate (CER). *Right scale:* ethanol and feed-rate. The amount of formed biomass and the concentrations of HMF and furfural can be seen in Fig.2.
Fig.2. Batch and fed-batch fermentations with *A:* CBS 8066, *B:* Baker's yeast, *C:* TMB3000, *D:* TMB3006. The CER, feed-rate and amount of formed ethanol from these experiments can bee seen in Fig.1. *Top row:* Batch fermentations where hydrolyzate was added with maximal rate (approximately 2000 ml/h). *Bottom row:* Fed-batch where the feed-rate was controlled by the program previously developed (see Materials and Methods). *Left scale:* biomass. *Right scale:* HMF and furfural concentrations.
Fig.3. Fermentations with TMB3006. *A:* Batch fermentation where 1.5 liter of hydrolyzate with maximum feeding rate of approximately 2000 ml/h. *B:* Fed-batch with the previously developed control strategy. *C:* Fed-batch fermentation with a low feed-rate. 17 hours after the start of the initial batch the feed-rate is set to 25 ml/h. At 48 hours the feed-rate is decreased to 12.5 ml/h until totally 850 ml of hydrolyzate has been added. *Right scale:* xylose and xylitol concentration. *Left scale:* xylose consumption.
Fig.4. Enzyme activity measurements from fed-batches with CBS 8066 (white bars) and TMB3000 (gray bars). *A:* Enzyme activity for conversion of furfural. *B:* Enzyme activity for the conversion of HMF. *Top row:* NADH used as the co-factor. *Bottom row:* NADPH used as the co-factor. Time = 0 h corresponds to the start of the fed-batch phase.
Fig. 5. ADH activity measured in fed-batch fermentations with CBS 8066 (white bars) and TMB3000 (gray bars). Time = 0 h corresponds to the start of the fed-batch phase.
Fig. 6 Array expression for different genes. Black bars: mRNA from TMB3000, Striped bars: mRNA from TMB3000 grown on synthetic media supplemented with 0.5 g/l HMF, Grey bars: mRNA from CBS8066, White bars: mRNA from CBS8066 grown on synthetic media supplemented with 0.5 g/l HMF
Fig. 7 Enzymatic conversion rates of cell free extract from the Exclone collection over expressing different genes. Black bars: conversion rate of furfural with NADH, Striped bars: conversion rate of furfural with NADPH, Grey bars: conversion rate of HMF with NADH, White bars: conversion rate of HMF with NADPH.
Fig. 8 Specific ethanol oxidation activity (in mU/mg protein) from cell extracts using NAD⁺ as cofactor. 113-5D=CEN.PK 113-5D with empty vector YEplac-HXT, C/ADH6-m= clone m with *ADH6* gene from CEN.PK strain overexpressed, T/ADH6-n= clone n with *ADH6* gene from TMB3000 strain overexpressed.
Fig. 9 Specific enzyme activities in crude cell extracts for the control strain CEN.PK113-5D (YEplac-HXT) and the ADH6-overexpressing strains C/ADH-2 and T/ADH6.2. *A:* Conversion of furfural with NADH as co-factor. *B:* Conversion of HMF with NADH as the co-factor. *C:* Conversion of furfural with NADPH as the co-factor. *D:* Conversion of HMF with NADPH as the co-factor.
Fig. 10 Specific DHAP reduction activity in crude cell extracts for the CEN.PK113-5D (YEplacHXT) control strain, the *ADH6*-overexpression strains C/ADH6-2 and T/ADH6-2 and strain TMB3000, using NADH (A) and NADPH (B) as co-factor.
Fig. 11 Batch fermentation of a dilute-acid hydrolyzate with the control strain CEN.PK113-5D (YEplacHXT). An arrow indicates the addition of hydrolyzate.
Fig. 12 Batch fermentation of a dilute-acid hydrolyzate with strain T/ADH6-2. The arrow indicates the addition of hydrolyzate.

## Claims

1. An ethanol producing *Saccharomyces cerevisiae* strain CEN.PK113-5D, being able to grow and produce ethanol from lignocellulosic hydrolysates comprising growth inhibiting compounds of the group furfural and 5-hydroxy-methyl furfural, in a batch, fed-bacth or continous fermentation, which strain is upregulated and/or over expressed with regard to the ADH6 gene, wherein the alcoholdehydrogenase is NADPH dependent.

2. Use of an ethanol producing *Saccharomyces cerevisiae* strain for growing and producing ethanol from lignocellulosic hydrolysates comprising growth inhibiting compounds of the group furfural and 5-hydroxy-methyl furfural, in a batch, fed-bacth or continuous fermentation, which strain is upregulated and/or over expressed with regard to the ADH6 gene, wherein the alcoholdehydrogenase is NADPH dependent.

3. Use according to claim 2, wherein said *Saccharomyces cerevisiae* strain is CEN.PK113-5D.

4. Use according to claim 2 or 3, for the reduction of furfural.

## Patentansprüche

1. Ethanol erzeugender *Saccharomyces-cerevisiae*-Stamm CEN.PK 113-5D, welcher in einer Batch-, Fed-Batch- oder kontinuierlichen Vergärung aus Lignocellulose-Hydrolysaten, welche aufbauhemmende Verbindungen der Gruppe Furfural und 5-Hydroxymethylfurfural umfassen, Ethanol aufbauen und erzeugen kann, wobei der Stamm bezüglich des ADH6-Gens hinaufreguliert und/oder überexprimiert ist, wobei die Alkoholdehydrogenase NADPH-abhängig ist.

2. Verwendung eines Ethanol erzeugenden *Saccharomyces-cerevisiae-*Stamms zum Aufbauen und Erzeugen von Ethanol aus Lignocellulose-Hydrolysaten, welche aufbauhemmende Verbindungen der Gruppe Furfural und 5-Hydroxymethylfurfural umfassen, in einer Batch-, Fed-Batch- oder kontinuierlichen Vergärung, wobei der Stamm bezüglich des ADH6-Gens hinaufreguliert und/oder überexprimiert ist, wobei die Alkoholdehydrogenase NADPH-abhängig ist.

3. Verwendung nach Anspruch 2, wobei es sich bei dem *Saccharomyces-cerevisiae-*Stamm um CEN.PK 113-5D handelt.

4. Verwendung nach Anspruch 2 oder 3 zur Reduktion von Furfural.

## Revendications

1. Souche de *Saccharomyces cerevisiae* produisant de l'éthanol CEN.PK113-5D, capable de développement et de production d'éthanol à partir d'hydrolysats de lignocellulose comprenant des composés d'inhibition de la croissance du groupe du furfural et du 5-hydroxy-méthyl furfural, dans une fermentation classique, à écoulement discontinu, ou continue, le gène ADH6 étant régulé à la hausse et/ou surexprimé dans cette souche, l'alcool déshydrogénase étant NADPH dépendante.

2. Utilisation d'une souche de *Saccharomyces cerevisiae* produisant de l'éthanol pour le développement et la production d'éthanol à partir d'hydrolysats de lignocellulose comprenant des composés d'inhibition de la croissance du groupe du furfural et du 5-hydroxy-méthyl furfural, dans une fermentation classique, à écoulement discontinu, ou continue, le gène ADH6 étant régulé à la hausse et/ou surexprimé dans cette souche, l'alcool déshydrogénase étant NADPH dépendante.

3. Utilisation selon la revendication 2, ladite souche de *Saccharomyces cerevisiae* étant CEN.PK113-5D.

4. Utilisation selon la revendication 2 ou la revendication 3, pour la réduction du furfural.
